# EUROPEAN PATENT APPLICATION

(11) **EP 4 775 213 A1**
(43) Date of publication of application: **15.07.2026**
(21) Application number: 25817409.3
(22) Date of filing: 15.05.2025
(51) Int. Cl.: A61K 35/745, A61K 35/74, A61P 19/06, C12N 1/20, C12N 1/04, C12R 1/01, C12R 1/225

(54) **COMPOSITE PROBIOTIC FORMULATION FOR ALLEVIATING HYPERURICEMIA AND USE THEREOF**

(30) Priority: 04.09.2024 CN 202411232543
(71) Applicant: Wecare Probiotics Co., Ltd., Suzhou, Jiangsu 215200 (CN)
(72) Inventor: FANG, Shuguang, Suzhou, Jiangsu 215200 (CN); QI, Yongmei, Suzhou, Jiangsu 215200 (CN); DONG, Yao, Suzhou, Jiangsu 215200 (CN); GAI, Zhonghui, Suzhou, Jiangsu 215200 (CN); ZHU, Jianguo, Suzhou, Jiangsu 215200 (CN)
(86) International application number: PCT/CN2025/095105
(87) International publication number: WO 2026/051421

(57) **Abstract**

The present invention involves a compound probiotic preparation for alleviating hyperuricemia and use thereof, and the strains in the compound probiotic preparation for alleviating hyperuricemia include the Bifidobacterium animalis subsp. lactis BLa80 strain with a collection No. of CGMCC No. 15410, the Lactobacillus rhamnosus LRa05 strain with a collection No. of CGMCC No.1.12734, and the Bifidobacterium breve BBr60 strain with a collection No. of CGMCC No.12915. The three strains can cooperate with each other for mutual promotion, thus synergizing the effect of alleviating hyperuricemia. This is specifically manifested in the following aspects: alleviating the elevated uric acid caused by hyperuricemia, and reducing xanthine oxidase activity and serum uric acid nitrogen level.

## Description

### Technical Field

The present invention belongs to technical field of probiotics, and involves compound probiotic preparation for alleviating hyperuricemia and use thereof.

### Background Art

As a major risk factor for gout, hyperuricemia is closely related to the occurrence of a variety of diseases including hypertension, diabetes, cardiovascular disease and kidney disease, and has become one of the public health problems that endanger people's life safety. Uric acid, the end product of purine metabolism, is mostly excreted through the kidneys and intestines. At present, the main conventional treatment method for hyperuricemia is medication, e.g., uric acid synthesis inhibitors and uric acid excretion promoters. However, these drugs may have side effects or adverse reactions, e.g., gastrointestinal discomfort, skin rash, hepatic or renal function damage, etc., and some patients have different responses to these drugs.

In recent years, with the in-depth research on intestinal microecology, scientists have found that probiotics play an important role in regulating the health of human body and promoting the metabolism of uric acid, thus providing new ideas for the treatment of hyperuricemia. As active microorganisms that are beneficial to the human body, probiotics play an important role in maintaining the balance of intestinal flora, promoting the absorption of nutrients, and enhancing the immunity. In recent years, several studies have shown that probiotics can affect uric acid metabolism through a variety of mechanisms. When probiotics grow and proliferate in the intestine, they absorb purine substances for use in the synthesis of their own genetic genes and degrade purine substances in the intestinal tract through producing purine nucleosidase, thereby reducing the conversion of purine substances into uric acid. Probiotics can enrich the intestinal flora, inhibit the growth of harmful bacteria, restore the intestinal barrier function, and thus improve the intestinal environment for uric acid metabolism. Probiotics can promote the uric acid excretion and reduce the serum uric acid level through affecting the expression of urate transport protein.

Although probiotics have shown great potential in alleviating hyperuricemia, considering the wide variety and uneven quality of probiotic products on the current market, it is important to develop more effective probiotic-based treatment regimens, which can not only reduce the uric acid level effectively, but also abate the demand for conventional medication.

### Contents of the Invention

In view of the deficiencies of the prior art, the present invention aims to provide compound probiotic preparation for alleviating hyperuricemia and use thereof, specifically relates to a compound probiotic preparation for alleviating hyperuricemia and its use in preparing drugs for the prevention, alleviation or treatment of hyperuricemia.

To attain the above object, the present invention employs the following technical solution:

In a first aspect, the present invention provides a compound probiotic preparation for alleviating hyperuricemia, and the strains in the compound probiotic preparation for alleviating hyperuricemia include the Bifidobacterium animalis subsp. lactis BLa80 strain with a collection No. of CGMCC No.15410, the Lactobacillus rhamnosus LRa05 strain with a collection No. of CGMCC No.1.12734, and the Bifidobacterium breve BBr60 strain with a collection No. of CGMCC No.12915.

For the present invention, a brand-new probiotic compounding method and a brand-new strategy for alleviating hyperuricemia have been creatively developed, that is, after the Bifidobacterium animalis subsp. lactis BLa80 strain, the Lactobacillus rhamnosus LRa05 strain and the Bifidobacterium breve BBr60 strain are compounded for combination use, it is found that the three can cooperate with each other for mutual promotion, thus synergizing the effect of alleviating hyperuricemia. This is specifically manifested in the following aspects: alleviating the elevated uric acid caused by hyperuricemia, and reducing xanthine oxidase activity and serum uric acid nitrogen level. In addition, all of the three strains are probiotics, so the product is safer and resistance to the product will not be easily generated.

Preferably, the viable content in the compound probiotic preparation is not less than 1×108 CFU/g or 1×108 CFU/mL, for example, the viable content is 1×108 CFU/g (CFU/mL), 1×109 CFU/g (CFU/mL), 1×1010 CFU/g (CFU/mL), 5×1010 CFU/g (CFU/mL), 1×1011 CFU/g (CFU/mL), 3×1011 CFU/g (CFU/mL), 5× 1011 CFU/g (CFU/mL), 1×1012 CFU/g (CFU/mL), 1×1013 CFU/g (CFU/mL), etc. Other specific values in the numeric range can also be selected, which will not be detailed here.

Preferably, the viable count ratio of the BLa80 strain, the LRa05 strain and the BBr60 strain is (1-10) :(1-10) :(1-10), wherein specific values in the range of (1-10) can be independently selected as 1, 2, 3, 4, 5, 6, 7, 8, 9, 10, etc., which will not be detailed here.

Preferably, the dosage forms of the compound probiotic preparation include solution, freeze-dried powder, capsules, tablets or granules. The dosage forms of the probiotic preparation in this invention are not limited, and include the most commonly used solution, freeze-dried powder, further-prepared capsules, tablets or granules.

Preferably, freeze-dried powder is adopted as the dosage form of the compound probiotic preparation, which is prepared by the following method:

Inoculate the BLa80 strain, LRa05 strain and BBr60 strain in the culture medium respectively for activation and fermentation culture in sequence, so as to obtain the fermentation broths. Centrifuge the fermentation broths respectively to obtain bacterial sludges. Mix the bacterial sludges with a protective agent, and resuspend to obtain resuspensions. Freeze-dry the resuspensions to obtain BLa80 bacterial powder, LRa05 bacterial powder and BBr60 bacterial powder. Mix the BLa80 bacterial powder, LRa05 bacterial powder and BBr60 bacterial powder according to the viable count ratio to obtain the compound probiotic preparation.

Preferably, the protective agent is composed of any one or at least two of skim milk, gelatin, dextrin, arabic gum, dextran, sodium alginate, polyvinylpyrrolidone, sucrose, lactose, trehalose, sorbitol, and xylitol.

Preferably, the temperature for fermentation culture is in the range of 35 to 40 °C, for example, 35 °C, 36 °C, 37 °C, 38 °C, 39 °C, 40 °C, etc. The duration is in the range of 12h to 30h, for example, 12h, 15h, 16h, 18h, 20h, 24h, 28h, 30h, etc. Other specific values in the above numeric ranges can also be selected, which will not be detailed here.

Preferably, the dose for the inoculation is in the range of 1 to 5%, for example, 1%, 2%, 3%, 4%, 5%, etc. Other specific values in the numeric range can also be selected, which will not be detailed here.

Preferably, MRS medium is used as the culture medium.

In a second aspect, the present invention provides the use of the compound probiotic preparation described in the first aspect in the preparation of drugs for the prevention, alleviation or treatment of hyperuricemia.

Preferably, the drugs also contain excipients.

Preferably, the excipients include any one or at least two of fillers, binders, wetting agents, disintegrants, emulsifiers, co-solvents, solubilizers, osmotic pressure regulators, colorants, pH adjusters, antioxidants, bacteriostatic agents or buffers.

Compared with the prior art, the present application has the following beneficial effects:
For the invention, a brand-new probiotic compounding method and a brand-new strategy for alleviating hyperuricemia have been creatively developed, that is, after the Bifidobacterium animalis subsp. lactis BLa80 strain, the Lactobacillus rhamnosus LRa05 strain and the Bifidobacterium breve BBr60 strain are compounded for combination use, it is found that the three can cooperate with each other for mutual promotion, thus synergizing the effect of alleviating hyperuricemia. This is specifically manifested in the following aspects: alleviating the elevated uric acid caused by hyperuricemia, and reducing xanthine oxidase activity and serum uric acid nitrogen level.

According to the Classified Nomenclature, the BLa80 strain involved in the present invention is named as Bifidobacterium animalis subsp. lactis, which was collected by the China General Microbiological Culture Collection Center on March 5, 2018, under the collection No. of CGMCC No. 15410, at the address: No. 3, Yard 1, Beichen West Road, Chaoyang District, Beijing.

According to the Classified Nomenclature, the LRa05 strain involved in the present invention is named as Lactobacillus rhamnosus, which was collected by the China General Microbiological Culture Collection Center on July 20, 2020, under the collection No. of CGMCC No.1.12734, at the address: No. 3, Yard 1, Beichen West Road, Chaoyang District, Beijing.

According to the Classified Nomenclature, the BBr60 strain involved in the present invention is named as Bifidobacterium breve, which was collected by the China General Microbiological Culture Collection Center on August 29, 2016, under the collection No. of CGMCC No.12915, at the address: No. 3, Yard 1, Beichen West Road, Chaoyang District, Beijing.

### Description of Drawings

Fig. 1 shows the statistical results of serum uric acid content on the 7th day of each group of rats.
Fig. 2 shows the statistical results of serum uric acid content on the 28th day of each group of rats.
Fig. 3 shows the statistical results of fecal uric acid content on the 28th day of each group of rats.
Fig. 4 shows the statistical results of fecal uric acid excretion on the 28th day of each group of rats.
Fig. 5 shows the statistical results of serum xanthine oxidase activity on the 28th day of each group of rats.
Fig. 6 shows the statistical results of serum urea nitrogen activity on the 28th day of each group of rats.

### Embodiments

The technical solution of the present disclosure will be further detailed below in embodiments. Those skilled in the art should appreciate that the examples described herein are only intended to assist in understanding of the present application, and should not be regarded as constituting any specific limitation on the present application.

The formulation of the medium involved in the following examples are as follows:
MRS medium: peptone 10g/L, beef extract 10g/L, glucose 20g/L, sodium acetate 2g/L, yeast powder 5g/L, diammonium hydrogen citrate 2g/L, K2PO4·3H2O 2.6g/L, MgSO4·7H2O 0.1g/L, MnSO4 0.05g/L, Tween 80 1mL/L, cysteine hydrochloride 0.5g/L.

According to the Classified Nomenclature, the BLa80 strain involved in the following examples is named as Bifidobacterium animalis subsp. lactis, which was collected on March 5, 2018, under the collection No. of CGMCC No.15410.

According to the Classified Nomenclature, the LRa05 strain involved in the following example is named as Lactobacillus rhamnosus, which was collected on July 20, 2020, under the collection No. of CGMCC No.1.12734.

According to the Classified Nomenclature, the BBr60 strain involved in the following example is named as Bifidobacterium breve, which was collected on August 29, 2016, under the collection No. of CGMCC No.12915.

Preparation method for the probiotic powder mentioned below: inoculated the seed solutions of BLa80 strain, LRa05 strain and BBr60 strain in MRS medium respectively, with the inoculation dose of each strain is 3% of the total mass of the culture medium; incubated at 37 °C for 18h, ot obtain the culture solution, then centrifuge the culture solution to obtain the bacterial cells; resuspend the bacterial cells with freeze-dried protective agent (aqueous trehalose solution with a concentration of 100g/L), wherein mass ratio of freeze-dried protective agent to the bacterial cells is 2:1, then obtain resuspension solution. Freeze-dry the resuspension solution by a vacuum-freezing process to obtain freeze-dried powders of the three strains. Mix two or three of the BLa80 freeze-dried powder, LRa05 freeze-dried powder and BBr60 freeze-dried powder based on the viable count ratio.

The test results are statistically analyzed using ggplot2 in R language. For a companion with the control group, ### represents p<0.001, ## represents p<0.01, # represents p<0.05, and for a companion with the model group, *** represents p<0.001, ** represents p<0.01, * represents p<0.05.

### Examples

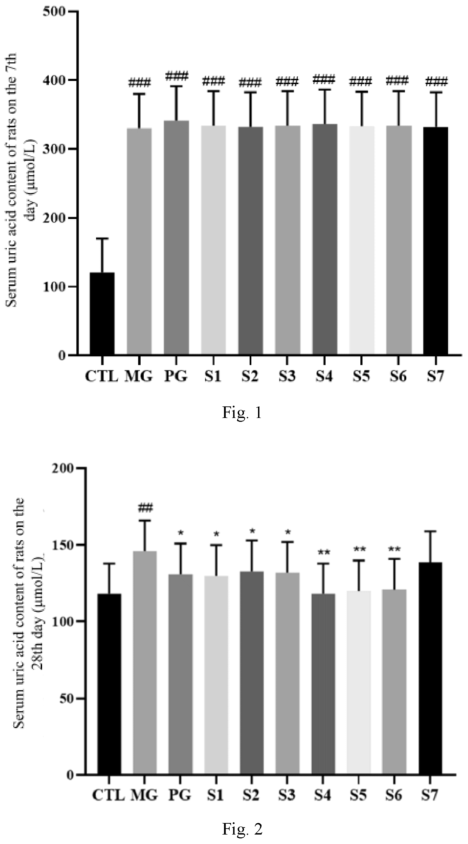

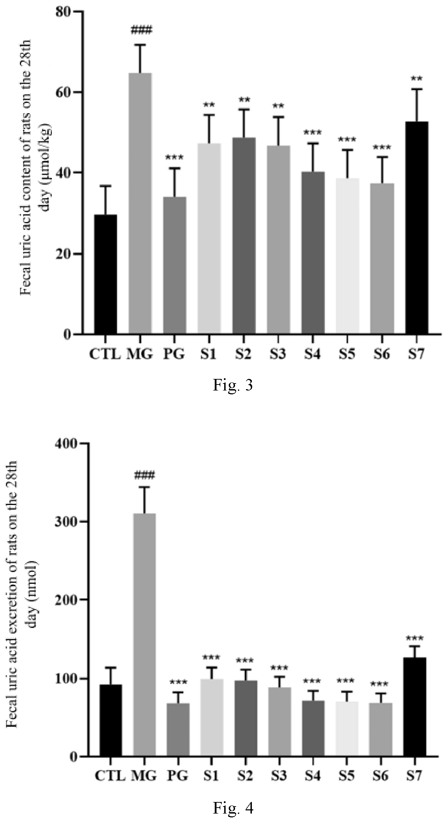

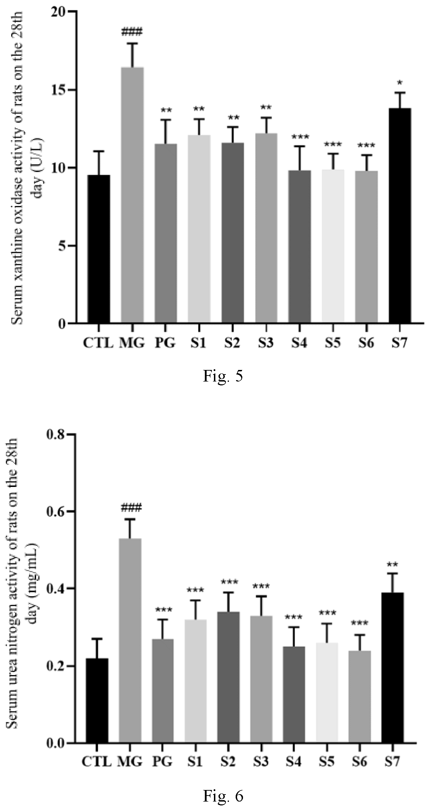

This example is to explore the ability of the compound probiotic preparation to improve the symptoms of rat model with hyperuricemia:
(1) Test animals: Healthy SPF grade male SD rats, 7 weeks old (80 rats). The rats were raised in a controlled environment under a 12h light/dark cycle, room temperature: 22-26 °C, humidity: 50-60%. The animals could eat and drink freely.
(2) Animal grouping: After 1 week of adaptive feeding, the rats were randomly divided into 10 groups with 8 rats in each group: control group (CTL), high uric acid model group (MC group), allopurinol group (PG group, positive control), S1 group (combined intervention with BLa80 freeze-dried powder and LRa05 freeze-dried powder, viable count ration: 1:1), S2 group (combined intervention with BLa80 freeze-dried powder and BBr60 freeze-dried powder, viable count ratio: 1:1), S3 group (combined intervention with LRa05 freeze-dried powder and BBr60 freeze-dried powder, viable count ration: 1:1), S4 group (combined intervention with BLa80 freeze-dried powder, LRa05 freeze-dried powder and BBr60 freeze-dried powder, viable count ratio: 1:1:1), S5 group (combined intervention with BLa80 freeze-dried powder, LRa05 freeze-dried powder and BBr60 freeze-dried powder, viable count ratio: 10:10:1), S6 group (combined intervention with BLa80 freeze-dried powder, LRa05 freeze-dried powder and BBr60 freeze-dried powder, viable count ratio: 1:1:10), and S7 group (combined intervention with commercially available Bifidobacterium animalis subsp. lactis ATCC700541 freeze-dried powder, commercially available Lactobacillus rhamnosus ATCC53103 freeze-dried powder and commercially available Bifidobacterium breve ATCC15700 freeze-dried powder, viable count ratio: 1:1:1).
(3) Animal modeling and intervention methods:
   The groups were fed with rat food containing 2.5% uric acid and intragastrically administered oteracil potassium at a dosage of 750mg/kg body weight every day, except for the control group. On the 7th day of the experiment, blood was collected from the orbital vein after anesthesia with ether, so as to verify whether the model was successfully established through measuring the level of uric acid in the blood. Subsequently, from the 8th day, the probiotic intervention groups were intragastrically administered solutions of probiotic freeze-dried powders (with normal saline as the solvent) at a dosage of 2.5×109 CFU/kg body weight every day, and the allopurinol group was intragastrically administered allopurinol (with normal saline as the solvent) at a dosage of 30 mg/kg body weight, while the control group and model group were given the same volume of normal saline.
(4) Sample collection:
   The experiment lasted until the 28th day, then the rats were anesthetized with ether again and blood was taken to obtain a serum samples, and at the same time, fecal samples of rats were collected. An automatic biochemical analyzer was used to detect the content of uric acid in serum and feces, as well as the content of urea nitrogen in serum. The activity of xanthine oxidase in serum was determined by using the xanthine oxidase activity assay kit. The fecal uric acid excretion was calculated as follows: fecal uric acid excretion = fecal uric acid concentration × weight of feces.
(5) Analysis of indicators:
   (5.1) Changes in serum uric acid level in rats on the 7th day:
      As shown in Fig. 1, after consecutive 7 days of oteracil potassium intragastric administration in combination with high uric acid diet, compared with the control group, the serum uric acid level in rats of the other groups increased significantly, which verified the successful establishment of the hyperuricemia model.
   (5.2) Changes in serum uric acid level in rats on the 28th day:
      After consecutive 21 days of intervention, as shown in Fig. 2, compared with the high uric acid model group, the serum uric acid level in rats decreased to varying degrees in all probiotic intervention groups and allopurinol intervention group, wherein, the effect of combined intervention of BLa80 freeze-dried powder, LRa05 freeze-dried powder and BBr60 freeze-dried powder was better than that of other compound formulas.
   (5.3) Changes in fecal uric acid content in rats on the 28th day:
      After consecutive 21 days of intervention, as shown in Fig. 3, compared with the high uric acid model group, the fecal uric acid content in rats decreased to varying degrees in all probiotic intervention groups and allopurinol intervention group, wherein, the effect of combined intervention of BLa80 freeze-dried powder, LRa05 freeze-dried powder and BBr60 freeze-dried powder was more significant.
   (5.4) Changes in fecal uric acid excretion in rats on the 28th day:
      After consecutive 21 days of intervention, as shown in Fig. 4, compared with the high uric acid model group, the fecal uric acid excretion in rats decreased to varying degrees in all probiotic intervention groups and allopurinol intervention group, wherein the combined intervention of BLa80 freeze-dried powder, LRa05 freeze-dried powder and BBr60 freeze-dried powder was more effective in reduction of uric acid excretion.
      The immediate and long-term effects of the interventions on uric acid metabolism were comprehensively evaluated by measuring fecal uric acid content and excretion, and thus, the regulatory effect of the interventions on the pathway of uric acid excretion of rats was understood more accurately.
   (5.5) Changes in serum xanthine oxidase activity in rats on the 28th day:
      As shown in Fig. 5, the test results of serum xanthine oxidase activity showed that hyperuricemia led to a significant increase of serum xanthine oxidase activity in rats as compared with the control group. On the 28th day, the xanthine oxidase activity of probiotic intervention groups and allopurinol intervention group was significantly lower than that of the high uric acid model group, indicating that the probiotic preparation involved in the present invention can inhibit the serum xanthine oxidase activity in rats with hyperuricemia, and the effect of the three-strain compound formula is better than that of other compound formulas.
   (5.6) Changes in serum urea nitrogen activity in rats on the 28th day:
      As shown in Fig. 6, on the 28th day, the serum urea nitrogen level of the rats in the high uric acid model group was significantly higher than that in the control group, indicating that hyperuricemia can cause inflammatory response and impaired renal function in rats. The urea nitrogen content of probiotic intervention groups was lower than that of the model group, reflecting that the probiotic preparation involved in the present invention can help alleviate kidney damage in rats with hyperuricemia, and in terms of reducing urea nitrogen, the effect of the three-strain compound formula is better.

The applicant states: the technical solution of the present invention is illustrated through the above examples, but the present application is not limited to those examples, which is to say, the present invention can be implemented without relying on the above-mentioned examples. Those skilled in the art should appreciate that any improvement made to the present invention, equivalent replacement of raw materials and addition of auxiliary components in the products of the present invention, or selection of specific methods, etc. fall within the scope of protection and disclosure of the present invention.

The above provides a detailed description of the preferred embodiment of the present invention, however, the invention is not limited to the specific details mentioned in the above-mentioned embodiments. Those skilled in the art can make several simple modifications to the technical scheme of the present invention, without departing from the spirit of the present invention. All those modifications shall be deemed as falling into the protection scope of the present invention.

In addition, it should be noted that the specific technical features described in above embodiments can be combined in any appropriate form, provided that there is no conflict. In order to avoid unnecessary repetition, the present invention will not elaborate on all possible combinations any further.

## Claims

1. A compound probiotic preparation for alleviating hyperuricemia, **characterized in that** the strains in the compound probiotic preparation for alleviating hyperuricemia include the Bifidobacterium animalis subsp. lactis BLa80 strain with a collection No. of CGMCC No.15410, the Lactobacillus rhamnosus LRa05 strain with a collection No. of CGMCC No.1.12734, and the Bifidobacterium breve BBr60 strain with a collection No. of CGMCC No.12915.

2. The compound probiotic preparation according to claim 1, **characterized in that** the viable content in the compound probiotic preparation is not less than 1×108 CFU/g or 1×108 CFU/mL.

3. The compound probiotic preparation according to claim 1, **characterized in that** the viable count ratio of the BLa80 strain, the LRa05 strain and the BBr60 strain is (1-10) :(1-10) :(1-10).

4. The compound probiotic preparation according to claim 1, **characterized in that** the dosage forms of the compound probiotic preparation include solution, freeze-dried powder, capsules, tablets or granules.

5. The compound probiotic preparation according to claim 4, **characterized in that** freeze-dried powder is adopted as the dosage form of the compound probiotic preparation, which is prepared by the following method: inoculate the BLa80 strain, LRa05 strain and BBr60 strain in the culture medium respectively for activation and fermentation culture in sequence, to obtain the fermentation broths; centrifuge the fermentation broths respectively to obtain bacterial sludges; mix the bacterial sludges with a protective agent respectively, and resuspend the mixtures to obtain resuspension solutions respectively; freeze-dry the resuspension solutions to obtain BLa80 bacterial powder, LRa05 bacterial powder and BBr60 bacterial powder respectively; mix the BLa80 bacterial powder, LRa05 bacterial powder and BBr60 bacterial powder according to the viable count ratio to obtain the compound probiotic preparation.

6. The compound probiotic preparation according to claim 5, **characterized in that** the protective agent is composed of any one or at least two of skim milk, gelatin, dextrin, arabic gum, dextran, sodium alginate, polyvinylpyrrolidone, sucrose, lactose, trehalose, sorbitol, and xylitol.

7. The compound probiotic preparation according to claim 5, **characterized in that** the temperature for fermentation culture is in the ranged of 35 to 40 °C, and the duration is in the range of 12 to 30h; preferably, the dose for the inoculation is in the range of 1 to 5%; preferably, MRS medium is used as the culture medium.

8. Use of the compound probiotic preparation according to any one of claims 1-7 in the preparation of drugs for the prevention, alleviation or treatment of hyperuricemia.

9. The use according to claim 8, **characterized in that**, the drugs also contain excipients.

10. The use according to claim 9, **characterized in that** the excipients include any one or at least two of fillers, binders, wetting agents, disintegrants, emulsifiers, co-solvents, solubilizers, osmotic pressure regulators, colorants, pH adjusters, antioxidants, bacteriostatic agents or buffers.
